# EUROPEAN PATENT SPECIFICATION

(11) **EP 1 494 749 B1**
(45) Date of publication and mention of the grant of the patent: **08.11.2006**
(21) Application number: 03710082.3
(22) Date of filing: 04.04.2003
(51) Int. Cl.: A61K 31/4525, A61K 31/496, A61P 31/18

(54) **PROCESS FOR PREPARATION OF PHARMACEUTICAL COMPOSITION CONTAINING ANTIRETROVIRAL PROTEASE INHIBITOR WITH IMPROVED BIOAVAILABILITY**
VERFAHREN ZUR HERSTELLUNG EINER EINEN ANTIRETROVIRALEN PROTEASEHEMMER ENTHALTENDEN PHARMAZEUTISCHEN ZUSAMMENSETZUNG MIT VERBESSERTER BIOVERFÜGBARKEIT
PROCEDE DE PREPARATION D'UN COMPOSITION PHARMACEUTIQUE CONTENANT UN INHIBITEUR DE PROTEASE ANTIRETROVIRAL AYANT UNE BIODISPONIBILITE AMELIOREE

(30) Priority: 04.04.2002 IN MU03252002
(43) Date of publication of application: 12.01.2005
(73) Proprietor: Cadila Pharmaceuticals Limited, Bhat, Ahmedabad 382 210 (IN)
(72) Inventor: Khamar, Bakulesh, Ellisbridge, 380006 Ahmedabad (IN)
(74) Representative: Towler, Philip Dean
(86) International application number: PCT/IB2003/001222
(87) International publication number: WO 2003/084462

(56) References cited:
- EP-A1- 0 709 098
- WO-A1-96/25939
- US-A- 6 017 932

## Description

The present invention relates to pharmaceutical compositions containing antiretroviral protease inhibitor with improved bioavailability.

Antiretroviral protease inhibitors are one of the potent drugs in the management of HIV. Human immunodeficiency virus infection is a new epidemic affecting a large number of individuals across the globe. It typically affects young individuals and is a major cause of morbidity and mortality in young healthy individuals. This also affects the economies of various nations. There have been various drugs available up to now, broadly grouped as NRTI, NNRTI and protease inhibitors. All of them are effective against human immunodeficiency virus. They are used in combination. Some of these drugs are known to have a poor bioavailability and there is a need to improve bioavailability.

Currently available antiretroviral protease inhibitors and their dosage are as follows. Dosage forms of currently available antiretroviral protease inhibitors include:

| | | | |
|---|---|---|---|
| 1 | Saquinavir | Hard gelatin capsule | 200 mg |
| | | Soft Gelatin capsule | 200 mg |
| 2 | Indinavir | Capsule | 400 mg |
| | | | 200 mg |
| 3 | Nelfinavir | Tablet | 250 mg |
| | | | 50 mg |
| | | Oral powder | 15 mg/g |
| 4 | Amprenavir | Capsule | 150 mg |
| | | | 50 mg |
| | | Oral solution | 15 mg/ml |

Recommended Adult dosages for currently available antiretroviral protease inhibitors:

| | | Total per Dosage | Frequency | Total per day |
|---|---|---|---|---|
| 1 | Saquinavir | 1200 mg | 8 Hourly | 3600 mg |
| | | (6 capsules) | | (18 capsules) |
| 2 | Indinavir | 800mg | 8 Hourly | 2400 mg |
| | | (2 capsules) | | (6 capsules) |
| 3 | Nelfinavir | 750mg | 8 Hourly | 2250 mg |
| | | (3 tablets) | | (9 capsules) |
| 4 | Amprenavir | 1200 mg | 12 Hourly | 2400 mg |
| | | (8 capsules) | | (16 capsules) |
| 5 | Lopinavir | 400 mg | 8 Hourly | 1200 mg |
| | | (3 capsules) | | (9 capsules) |

From the above it is obvious that the patient has to consume a large number of pharmaceutical compositions for the management of HIV, as more than one antiretroviral drugs are used at the same time. This results in a reduction in compliance from the patient. Thus it is important to reduce the number of dosage forms to be consumed by the patient. Improvement of bioavailability would also be helpful. There are many methods of increasing bioavailability of pharmaceutical composition. This involves changes in the design of molecule, using a pleomorphic form of an active pharmaceutical ingredient, the use of different excipients, etc.

The bioavailability of a drug is altered by food also, e.g. Administration of indinavir with a meal high in calories, fat, and protein (784 kcal, 48.6 g fat, 31.3 g protein) resultsin a 77% ± 8% reduction in AUC and an 84% ± 7% reduction Cₘₐₓ(n=10).

A literature survey revealed that the use of certain herbs for this purpose, e.g. Piper longum and Piper nigrum as adjuncts in the Indian System of medicine, dates back to the 6^{th} century AD and the 3^{rd} century BC (Charaka et al, Charak Samhita, 3^{rd} edn, Nirnaya Sagar Press Bombay, 1991 [in Sanskrit]; Kaviraj, KB. Sushruta Samhita, 2^{nd}ed., Chowk hamba Sanskrit Series, voL 3, Varanasi, India 1953; Vagbmat, Ashtang Hridaya, Chowkhamba Sanskrit Series, Varansi, India, 1962 [in Sanskrit]).

Bose (Bose K.G., Pharmacopoeia Indica, Bose Laboratories, Calcutta, India 1928) makes a positive mention of the property of long pepper for increasing efficacy of Vasaka as an antiasthmatic.

In an attempt to study the scientific use of the above herbs, a research group (Usha Zutshi and J.L. Koul, Indian Drugs, 19 (12), 476479, 1982), observed the effect of Trikatu ( a composition comprising of *Piper nigrum, Piper longum & Zingiber officinalis* in equal proportion w/w) as a whole on vasicine, resulting in the enhanced bioavailability (and therefore the activity) of this drug to a great extent. *Piper longum* and *piper nigrum* are almost equally effective, whereas ginger (*Zingiber officinalis*) alone has no significant such enhancing effect

US Patent 5439891 describes the use of piperine to improve bioavailability of Rifampicin.

US Patent 5616593 describes the use of piperine to improve bioavailability of substances used to treat disease of the cardiovascular system, the central nervous system, the gastrointestinal tract or the hemopoetic system. US Patents 5744161 and 5536506 describe its use for bioenhancement of nutritional products. US Patent 6017932 describes its use for bioenhancement of NSAID. US Patent 5383478 describes its use in cigarette to provide enhanced satisfactory.

Surprisingly, according to the present invention it is also observed that it is possible to improve the bioavailability of protease inhibitors used in the management of BEIV. Protease inhibitors used in the management of HIV include Saquinavir, Ritonavir, Indinavir, Nelfinavir, Lopinavir, Amprenavir. According to present invention it is possible to improve the bioavailability of protease inhibitors like Saquinavir, Indinavir, Nelfmavir, Lopinavir and Amprenavir, but it is not possible to improve the bioavailability of Ritonavir. However, according to the present invention it is not possible to improve the bioavailability of other drugs used in the management of HIV like Zidovudine, Stavudine, Lamivudine, Zalcitabine, Didanosine, Abacavir, etc.

In accordance with the present invention there is disclosed a pharmaceutical composition using piperine, its metabolites, structural analogues or isomers thereof along with at least one antiretroviral protease inhibitor. It involves incorporation of piperine in a small quantity to the pharmaceutical composition containing antiretroviral protease inhibitor.

An object of the present invention is to provide a pharmaceutical composition containing antiretroviral protease inhibitor with improved bioavailability.

A further object of present invention is to provide a pharmaceutical compositions containing antiretroviral protease inhibitors with improved effect so that amount of the drug to be consumed is reduced.

A further object of present invention is to provide a pharmaceutical compositions containing antiretroviral protease inhibitors so that the number of dosage forms to be consumed is minimized.

A further object of present invention is to provide pharmaceutical compositions containing antiretroviral protease inhibitors with increased duration of action so that the frequency of dosage is reduced.

### DETAILED DESCRIPTION OF THE INVENTION

In one aspect, the present invention provides a pharmaceutical composition comprising an antiretroviral protease inhibitor, piperine or an isomer thereof, and physiologically acceptable excipients, wherein the antiretroviral protease inhibitor is selected from indinavir, saquinavir, amprenavir, nelfinavir and lopinavir.

In another aspect, the present invention provides the use of said antiretroviral protease inhibitor and piperine or an isomer thereof in the manufacture of a pharmaceutical composition for simultaneous, separate or sequential administration in the treatment of HIV.

The incorporation of piperine, its metabolites or structural analogues or isomers thereof with antiretroviral protease inhibitor/s results in a synergistic composition having unexpected increased bioavailability of antiretroviral protease inhibitor/s. The antiretroviral protease inhibitors are selected from Indinavir, Saquinavir, Amprenavir, Nelfinavir, Lopinavir. It is also to be noted that piperine or its metabolites or structural analogues or isomers thereof have no pharmacological properties, but when incorporated with antiretroviral protease inhibitor/s this results in a synergistic effect on said antiretroviral protease inhibitor/s, in the form of enhanced activity and bio-availability thereof.

In a preferred embodiment of the invention the quantity of piperine may range from 0.2% to 10% by weight of the antiretroviral protease inhibitor/s.

In the compositions of the invention, the unit dose of the antiretroviral protease inhibitor is typically in the range of 1-80 mg/kg body weight, whilst that of the piperine or isomer thereof is typically in the range of 0.01-2 mg/kg body weight

The quantity of piperine typically required for this purpose ranges up to 3 0 mg per dosage form to be administered. Increasing the amount of piperine beyond this does not improve the bioavailability of the drug further. Like excipients used in pharmaceutical compositions, piperine is not found to have any therapeutic effect in management of HIV when used in this amount. It is also not found to affect the chemical composition of a drug when combined with it in a single pharmaceutical composition. Since piperine does not have any effect on the chemical composition of the drug, it can be incorporated into any dosage form using techniques known to those skilled in the art.

Piperine, (*E,E*)-1-[5-(1,3-benzodioxyl 5-yl)-1-oxo-2,4-pentadenyl] piperidine, is the main constituent of many Piper species. It is mostly obtained from Piper longum (3-5%) or Piper nigrum (3-9%) which are cultivated commercially.

Piperine as a parent molecule and / or one or more of its metabolites and / or analogues thereof may have a role in enhancing bio-availability of drugs. Examples of piperine analogues are derivatives in which the piperidine ring is substituted, e.g. by an amino group, or esters (e.g. C₁-C₆ alkyl esters) of metabolites containing an OH group.

Piperine forms monoclinic prisms from ethanol, m.p. 130°C. It is tasteless at first but induces a burning sensation after a few seconds. It is neutral to litmus (pea 12.22). It is soluble in benzene, chloroform, ether, ethyl acetate, dichloromethane, alcohol, acetic acid and insoluble in water, pet. ether.

On alkaline hydrolysis it furnishes a base, piperidine, and the acid, viz. pierce acid, m.p. 216°C. It may be synthesized by condensing the two components under proper conditions.

It has the following spectral characteristics:
UV (methanol): max 340 mud (32,000).
IR(Kerr): 1633,1610,15 80,1510,1440,1250,1190,1130,1030,995,930,842 cm¹⁻.

Piperine is commercially available. In addition, piperine can be isolated from oleo-resin of Piper nigrum (Black pepper) or Piper longum (Long pepper). The powdered fruits of the plant (Piper nigrum) are extracted with dichloromethane at room temperature with stirring for 12 hours. The extract is filtered, concentrated in vacuum and the residue is subjected to purification on an alumina column. Piperine can be obtained either from petroleum ether/ethyl acetate fractions or dichloromethane to give crude piperine. Pure piperine can be obtained by crystallization from ethanol. Piperine can also be obtained directly from the crude residue in lesser amounts by extraction in alcohol, filtration and successive crystallization. Piperine metabolites, analogues and isomers can be prepared synthetically.

However, it is to be noted that the identical effect of the present invention is not seen when Ritonavir, an antiretroviral protease inhibitor, is incorporated with piperine. The bioavailability of Ritonavir containing pharmaceutical compositions made as per the present invention is not found to be significantly different compared to conventional compositions.
1. The following are examples of various pharmaceutical compositions containing antiretroviral protease inhibitor and piperine in accordance with the present invention. However, the scope of this invention is not limited by them.
Examples:

| | | |
|---|---|---|
| I | Amprenavir | 50 mg |
| | Piperine | 2 mg |
| | (Capsule) | |
| | | |
| II | Amprenavir | 150 mg |
| | Piperine | 3 mg |
| | (Capsule) | |
| | | |
| III | | per ml of solution |
| | Amprenavir | 15 mg |
| | Piperine | 1.0 mg |
| | | |
| IV | Lopinavir | 133.3 mg |
| | Piperine | 5.0 mg |
| | (Capsule) | |
| | | |
| V | Lopinavir Solution | per ml of solution |
| | Lopinavir | 80.0 mg |
| | Piperine | 3.0 mg |
| | | |
| VI | Saquinavir | 200 mg |
| | Piperine | 5 mg |
| | (Capsule) | |
| | | |
| VII | Nelfinavir | 250 mg |
| | Piperine | 10 mg |
| | (Tablet) | |
| | | |
| VIII | Nelfinavir | 50 mg |
| | Piperine | 3 mg |
| | (Tablet) | |
| | | |
| IX | Indinavir | 400 mg |
| | Piperine | 10 mg |
| | (Capsule) | |
| | | |
| X | Indinavir | 200 mg |
| | Piperine | 5 mg |
| | (Capsule) | |

2. Kit
In a further aspect, the present invention provides a kit comprising one or more pharmaceutically acceptable doses of an antiretroviral protease inhibitor as defined above and one or more pharmaceutically acceptable doses of piperine or an isomer thereof. They are supplied as single unit The two or more pharmaceutical compositions incorporated in the kit are in the form of liquid, powder, capsule, tablet and/or any combination thereof The amount of antiretroviral protease inhibitor included in the kit is adequate to provide therapeutic effect when consumed.
The following are Examples of kits containing antiretroviral protease inhibitor with improved bioavailability. However, the scope of this invention is not limited by them.
Indinavir
- i): A capsule containing 400 mg of Indinavir and a tablet containing 10 mg of piperine.
- ii): Two capsules each containing 400 mg of Indinavir and two tablets each containing 10 mg of piperine.
- iii): Two capsules containing 400 mg and 200 mg of Indinavir respectively and a tablet containing 20 mg of piperine.
Nelfinavir
- i): Two tablets each containing 250 mg of Nelfinavir and a tablet containing 20 mg of piperine.
- ii): Two tablets each containing 250 mg of Nelfinavir and two tablets each containing 15 mg of piperine.
Saquinavir
- i): Three capsules each containing 200 mg of Saquinavir and three tablets each containing 5 mg of piperine.
- ii): Four capsules each containing 200 mg of Saquinavir and a tablets containing 20 mg of piperine.

3. The following are Examples of excipients (i.e. other than piperine) which can be used to prepare pharmaceutical compositions of the present invention. However, the scope of this invention is not limited by them.
Saquinavir :
Capsule: medium chain mono- and diglycerides, povidone and dl-alpha tocopherol.
Nelfinavir:
Tablet: calcium silicate, crospovidone, magnesium stearate and FD&C blue # 2 powder. Oral powder: microcrystalline cellulose, maltodextrin, dibasic potassium phosphate, crosporvidone, hydroxypropyl methylcellulose, aspartame, sucrose palmitate, and natural and artificial flavour.
Amprenavir:
Capsule: d-Tocopheryl polyethylene glycol 1000 succinate (TPGS), polyethylene glycol 400 (PEG 400), and propylene glycol.
Lopinavir :
Capsule : FD&C Yellow No. 6, gelatin, glycerin. Oleic acid, polyoxyl 35 castor oil, propylene glycol, sorbitol special, titanium dioxide, and water.
Oral solution : Acesulfame potassium, alcohol, artificial cotton candy flavor, citric acid, glycerin, high fructose com syrup, Magnasweet-110 flavor, menthol, natural & artificial vanilla flavor, peppermint oil, polyoxyl 40 hydrogenated castor oil, povidone, propylene glycol, saccharin sodium, sodium chloride, sodium citrate, and water.
Indinavir:
Capsule: anhydrous lactose and magnesium stearate.

4. The following are illustrative Examples of the manufacture of various dosage forms. However, the scope of this invention is not limited by them.
Tablets:
Nelfinavir, piperine, calcium silicate, crospovidone, magnesium stearate and FD&C blue #2 powder are sifted through a 40 mesh sieve. The large sized particles are pulverized and sifted through the sieve again. The process is repeated again till all the material is sifted through the sieve. The material is blended and then granulated. The granules so obtained are compressed into tablets using rotary tablet manufacturing machine. The tablets so prepared are evaluated for quality control parameters and packed in appropriate packaging.
Capsules:
Indinavir, piperine, anhydrous lactose and magnesium stearate are provided after each of them is approved for its quality parameters. Each one of them is passed through a No. 20 mesh in a mechanical sifter. The oversized particles are pulverized in a multi mill at fast speed and sifted again. The powder so obtained is blended in an octagonal blender to obtain a blend which is ready for filling. The blend is transferred to capsule filling machine. Hard gelatin capsules are filled with the blend. They are checked for fill weight, amount of active per capsule and other quality control parameters before final packing.
Powder:
Nelfinavir, piperine, microcrystalline cellulose, maltodextrin, dibasic potassium phosphate, crospovideone, hydroxyprophyl methylcellulose, aspartame, sucrose palmitate are sifted through a No. 40 mesh sieve. The large sized particles are milled and sifted through the sieve again. The process is repeated again till all the material is passed through the sieve. The material is blended. The powder so obtained is evaluated for quality control parameters before packing.

5. Bioavailability:
The following example illustrates the improvement in bioavailability of the compositions of the present invention. The bioavailability of pharmaceutical composition made as per present invention is found to be markedly improved.
A) When the protease inhibitor Indinavir is incorporated in pharmaceutical compositions of the present invention there is a significant increase in Cₘₐₓ and AUC. There is a decrease in Tₘₐₓ with overall improvement in bioavailability. The bioavailability of Indinavir is found to be almost double that ordinarily found.
   The bioavailability of a single dose of Indinavir containing 400 mg of Indinavir was evaluated in a crossover design experiment in humans. When pharmacokinetic 400 mg of Indinavir conventionally available is compared with newly designed pharmaceutical composition containing 400 mg of Indinavir along with 10mg of piperine, it is found that bioavailability of novel composition is increased. The bioavailability of Indinavir made as per present invention having 400 mg of Indinavir along with 10 mg of piperine is found to be close to what can be achieved with 800 mg of Indinavir alone.
   When Indinavir was used in a group of healthy human volunteers, the mean Cₘₐₓ of the conventional formulation was found to be 2.0636, compared to 2.9213 of a composition of the present invention. Tₘₐₓ was found to improve from 1.58 to 1.05. The overall effect in AUC was an increase in AUC from 4.05 to 9.24.
B) The bioavailability of Ritonavir (also an antiretroviral protease inhibitor) is not altered when prepared in a manner similar to that of the present invention and so as per present invention it is not possible to improve bioavailability of Ritonavir.
In a crossover design experiment, conventional ritonavir as well as ritonavir in a composition analogous to those of the present invention were evaluated for their respective plasma concentration. Both the products gave identical plasma concentrations and no improvement was seen in the bioavailability of ritonavir in a composition analogous to those of the present invention. The AUC were found to be 5.53 and 6.29 respectively. This difference is not found to be significant.
6. Dosage:
Improved bioavailability of the drug results in alteration in the dosage to be taken by the patient. However, the scope of this invention is not limited by the following.
a) Pharmaceutical compositions of the present invention may be administered in a way identical (frequency and dosage) to the one determined by amount of antiretroviral protease inhibitor. When used in this pattern the plasma concentrations of antiretroviral protease inhibitor will be significantly higher than that observed with conventional pharmaceutical compositions.
b) Pharmaceutical compositions of the present invention may be administered at a frequency of administration determined by antiretroviral protease inhibitor present in the composition. When used in such a way the amount of antiretroviral protease inhibitor required to be administered at one time is significantly reduced to obtain identical plasma concentration.
c) Pharmaceutical compositions of the present invention may be administered in the same amount as if made conventionally. Under such circumstances desired plasma concentrations are maintained for a longer time and the frequency of administration of the antiretroviral protease inhibitor may be reduced.
d) Pharmaceutical compositions of the present invention may be administered in such a way that the amount of antiretroviral protease inhibitor used per day or at any one time is reduced along with frequency of administration.
e) Examples: The following are illustrative examples of the administration of novel compositions of the present invention in comparison to conventionally prepared and available compositions. However, the scope of the present invention is not restricted by them.

| | | |
|---|---|---|
| Drug | Conventional composition Amount /Frequency | Novel composition Amount /Frequency |
| Indinavir | 800 mg three times/day | 400 mg three times/day |
| | | 800 mg twice/day |
| | | 600 mg twice/day |
| Saquinavir | 1200 mg three times/day | 600 mg three times/day |
| | | 800 mg twice/day |
| | | 400 mg twice/day |
| Amprenavir | 1200 mg twice/day | 750 mg twice/day |
| Nelfinavir | 750 mg three times/day | 500 mg twice/day |

## Claims

1. A pharmaceutical composition comprising an antiretroviral protease inhibitor, piperine or an isomer thereof, and physiologically acceptable excipients, wherein the antiretroviral protease inhibitor is selected from indinavir, saquinavir, amprenavir, nelfinavir and lopinavir.

2. A pharmaceutical composition as claimed in claim 1 which further comprises any other drug(s).

3. A pharmaceutical composition as claimed in claim 1 or claim 2 which further comprises other antiretroviral protease inhibitor(s).

4. A pharmaceutical composition as claimed in any one of the preceding claims wherein the amount of piperine is from 0.1% to 10% by weight of the pharmaceutical composition.

5. A pharmaceutical composition as claimed in any one of the preceding claims wherein the amount of piperine is from 0.2% to 10% by weight of the antiretroviral protease inhibitor.

6. A pharmaceutical composition as claimed in any one of the preceding claims wherein the amount of piperine is less than 30 mg.

7. A pharmaceutical composition as claimed in any one of the preceding claims in the form of a liquid preparation, tablet, capsule or granule.

8. A pharmaceutical composition as claimed in any one of claims 1 to 6 in the form of a suspension of the active ingredients.

9. The use of an antiretroviral protease inhibitor and piperine or an isomer thereof in the manufacture of a pharmaceutical composition as defined in any one of claims 1 to 8 for simultaneous, separate or sequential administration of said antiretroviral protease inhibitor and piperine in the treatment of HIV, wherein said antiretroviral protease inhibitor is selected from indinavir, saquinavir, amprenavir, nelfinavir and lopinavir.

10. A kit comprising one or more pharmaceutically acceptable doses of an antiretroviral protease inhibitor and one or more pharmaceutically acceptable doses of piperine or an isomer thereof for use in treating HIV, wherein the antiretroviral protease inhibitor is selected from indinavir, saquinavir, amprenavir, nelfinavir and lopinavir.

11. A kit as claimed in claim 10 wherein the pharmaceutically acceptable doses are in the form of a liquid preparation, tablet, capsule or granule.

12. A kit as claimed in claim 10 wherein the pharmaceutically acceptable doses are in the form of a suspension of the active ingredients.

13. A kit as claimed in any one of claims 10 to 12 wherein said antiretroviral protease inhibitors and said piperine are for use simultaneously.

14. A kit as claimed in any one of claims 10 to 12 wherein said antiretroviral protease inhibitors and said piperine are for use sequentially.

15. A kit as claimed in any one of claims 10 to 12 wherein said antiretroviral protease inhibitors and said piperine are for use separately.

## Patentansprüche

1. Pharmazeutische Zusammensetzung, welche einen antiretroviralen Protease-Inhibitor, Piperine oder ein Isomer davon, und physiologisch akzeptable Arzneistoffträger umfasst, wobei der antiretrovirale Inhibitor aus Indinavir, Saquinavir, Amprenavir, Nelfinavir und Lopinavir ausgewählt wird.

2. Pharmazeutische Zusammensetzung nach Anspruch 1, welche ferner jegliche(s) andere(n) Arzneimittel umfasst.

3. Pharmazeutische Zusammensetzung nach Anspruch 1 oder 2, welche ferner (einen) andere(n) antiretrovirale(n) Protease-Inhibitor(en) umfasst.

4. Pharmazeutische Zusammensetzung nach einem der vorhergehenden Ansprüche, wobei die Menge des Piperine von 0,1% bis 10% Gewichtsprozent der pharmazeutischen Zusammensetzung beträgt.

5. Pharmazeutische Zusammensetzung nach einem der vorhergehenden Ansprüche, wobei die Menge des Piperine von 0,2% bis 10% Gewichtsprozent des antiretroviralen Protease-Inhibitors beträgt.

6. Pharmazeutische Zusammensetzung nach einem der vorhergehenden Ansprüche, wobei die Menge des Piperine weniger als 30 mg beträgt.

7. Pharmazeutische Zusammensetzung nach einem der vorhergehenden Ansprüche in der Form eines flüssigen Präparats, einer Tablette, einer Kapsel oder eines Körnchens.

8. Pharmazeutische Zusammensetzung nach einem der Ansprüche 1 bis 6 in der Form einer Suspension der Wirkstoffe.

9. Verwendung eines antiretroviralen Protease-Inhibitors und von Piperine oder eines Isomers davon bei der Herstellung einer pharmazeutischen Zusammensetzung, wie in einem der Ansprüche 1 bis 8 definiert ist, für die gleichzeitige, getrennte oder sequentielle Verabreichung des antiretroviralen Protease-Inhibitors und von Piperine bei der Behandlung von HIV, wobei der antiretrovirale Protease-Inhibitor aus Indinavir, Saquinavir, Amprenavir, Nelfinavir und Lopinavir ausgewählt wird.

10. Kit, der eine oder mehrere pharmazeutisch akzeptable Dosen eines antiretroviralen Protease-Inhibitors und eines oder mehrerer pharmazeutisch akzeptabler Dosen von Piperine oder eines Isomers davon zur Verwendung bei der HIV-Behandlung umfasst, wobei der antiretrovirale Protease-Inhibitor aus Indinavir, Saquinavir, Amprenavir, Nelfinavir und Lopinavir ausgewählt wird.

11. Kit nach Anspruch 10, wobei die pharmazeutisch akzeptablen Dosen in der Form eines flüssigen Präparats, einer Tablette, einer Kapsel oder eines Körnchens sind.

12. Kit nach Anspruch 10, wobei die pharmazeutisch akzeptablen Dosen in der Form einer Suspension der Wirkstoffe sind.

13. Kit nach einem der Ansprüche 10 bis 12, wobei die antiretroviralen Protease-Inhibitoren und das Piperine für eine gleichzeitige Verwendung vorgesehen sind.

14. Kit nach einem der Ansprüche 10 bis 12, wobei die antiretroviralen Protease-Inhibitoren und das Piperine für eine sequentielle Verwendung vorgesehen sind.

15. Kit nach einem der Ansprüche 10 bis 12, wobei die antiretroviralen Protease-Inhibitoren und das Piperine für eine getrennte Verwendung vorgesehen sind.

## Revendications

1. Composition pharmaceutique comprenant un inhibiteur de protéase antirétroviral, de la pipérine ou un isomère de celle-d, et des excipients physiologiquement acceptables, où l'inhibiteur de protéase antirétroviral est choisi parmi l'indinavir, le saquinavir, l'amprenavir, le nelfinavir et le lopinavir.

2. Composition pharmaceutique selon la revendication 1 qui comprend en outre un ou plusieurs autres médicaments quelconques.

3. Composition pharmaceutique selon la revendication 1 ou la revendication 2 qui comprend en outre un ou plusieurs autres inhibiteurs de protéase antirétroviraux.

4. Composition pharmaceutique selon l'une quelconque des revendications précédentes où la quantité de pipérine est de 0,1 % à 10 % par rapport à la masse de la composition pharmaceutique.

5. Composition pharmaceutique selon l'une quelconque des revendications précédentes où la quantité de pipérine est de 0,2 % à 10 % par rapport à la masse de l'inhibiteur de protéase antirétroviral.

6. Composition pharmaceutique selon l'une quelconque des revendications précédentes où la quantité de pipérine est inférieure à 30 mg.

7. Composition pharmaceutique selon l'une quelconque des revendications précédentes sous forme de préparation liquide, de comprimé, de capsule ou de granule.

8. Composition pharmaceutique selon l'une quelconque des revendications 1 à 6 sous forme d'une suspension des ingrédients actifs.

9. Utilisation d'un Inhibiteur de protéase antirétroviral et de pipérine ou d'un isomère de celle-d dans la fabrication d'une composition pharmaceutique telle que définie dans l'une quelconque des revendications 1 à 8 pour l'administration simultanée, séparée ou successive dudit Inhibiteur de protéase antirétroviral et de ladite pipérine dans le traitement du VIH, où ledit inhibiteur de protéase antirétroviral est choisi parmi l'indinavir, le saquinavir, l'amprenavir, le nelfinavir et le lopinavir.

10. Kit comprenant une ou plusieurs doses pharmaceutiquement acceptables d'un Inhibiteur de protéase antirétroviral et une ou plusieurs doses pharmaceutiquement acceptables de pipérine ou d'un isomère de celle-ci destiné à être utilisé dans le traitement du VIH, où l'inhibiteur de protéase antirétroviral est choisi parmi l'indinavir, le saquinavir, l'amprenavir, le nelfinavir et le lopinavir.

11. Kit selon la revendication 10 où les doses pharmaceutiquement acceptables sont sous la forme d'une préparation liquide, d'un comprimé, d'une capsule ou d'un granule.

12. Kit selon la revendication 10 où les doses pharmaceutiquement acceptables sont sous la forme d'une suspension des Ingrédients actifs.

13. Kit selon l'une quelconque des revendications 10 à 12 où lesdits inhibiteurs de protéase antirétroviraux et ladite pipérine sont destinés à être utilisés simultanément.

14. Kit selon l'une quelconque des revendications 10 à 12 où lesdits inhibiteurs de protéase antirétroviraux et ladite pipérine sont destinés à être utilisés successivement.

15. Kit selon l'une quelconque des revendications 10 à 12 où lesdits inhibiteurs de protéase antirétroviraux et ladite pipérine sont destinés à être utilisés séparément.
